(19) Europäisches Patentamt

European Patent Office

Office européen des brevets

(11) **EP 0 925 355 B1**

## (12) EUROPEAN PATENT SPECIFICATION

(45) Date of publication and mention
of the grant of the patent:
**02.01.2003 Bulletin 2003/01**

(21) Application number: **97939094.5**

(22) Date of filing: **11.09.1997**

(51) Int Cl.[7]: **C12M 1/00**

(86) International application number:
**PCT/GB97/02452**

(87) International publication number:
**WO 98/011199 (19.03.1998 Gazette 1998/11)**

(54) **APPARATUS FOR CULTIVATING MICROORGANISMS**

VORRICHTUNG ZUM KULTIVIEREN VON MIKROORGANISMEN

APPAREIL DE CULTURE DE MICRO-ORGANISMES

(84) Designated Contracting States:
**AT BE CH DE DK ES FI FR GB GR IE IT LI LU MC NL PT SE**

(30) Priority: **12.09.1996 GB 9619058**

(43) Date of publication of application:
**30.06.1999 Bulletin 1999/26**

(73) Proprietor: **Bioprocess AS**
**9850 Hirthals (DK)**

(72) Inventor: **NORSKER, Niels, Henrik**
**Bridge of Allen, Stirlingshire FK9 4NS (GB)**

(74) Representative: **Pattullo, Norman et al**
**Murgitroyd and Company**
**165-169 Scotland Street**
**Glasgow G5 8PL (GB)**

(56) References cited:
| | |
|---|---|
| WO-A-92/00380 | FR-A- 2 386 037 |
| US-A- 4 087 936 | US-A- 4 326 034 |
| US-A- 5 031 990 | US-A- 5 281 533 |

## Description

The invention relates to apparatus for cultivating microorganisms, and in particular algae.

Conventional microalgal cultivation for hatchery operations is usually done in plastic bags. The total production cycle is quite laborious. The long propagation procedure from pure stock cultures to large production bags introduces a significant risk that ciliates, other protozoans or microalgae may take over. Apparent evidence of such a situation is that the culture bleaches to "crash" soon after. Some species of ciliates may double several times a day- a growth rate which exceeds that of aquaculture algae, and accounts for the familiar experience that a bag may look fine one afternoon, and has crashed the next morning.

In some situations, production cultures are on the verge of such a crash. One may be able to harvest the culture just before the ciliates take over - but, if delayed, the crash is most likely to follow. Also suboptimum growth conditions for the algae make a successful harvest less likely.

With high cultivation volumes elevated temperature is very often a problem and limits the amount of light the culture may be given, especially in warm environments.

Closed semicontinuous microalgal cultures in the scale of 1-20 litres are on the other hand not difficult to maintain. Aseptically set up and regularly diluted with sterile growth medium, and sparged with air eventually containing a little $CO_2$, the culture may be continued for extended periods until attached growth on the walls of the flask makes change of flask necessary.

However, a disadvantage of this semicontinuous process is that it is labour intensive and wall growth limits its usefulness.

Often the so called flashing light principle is used to enhance photosynthetic yield in microbial cultures.

According to this principle, the photosynthetic energy conversion of a given amount of visible light in a culture of photosynthetic microorganisms such as micro algae, is increased when the light is administered in high intensity pulses, separated by longer intervals of darkness.

The principle yields photosynthetic energy conversion increments with light emitted in pulses of a duration as little as µseconds to minutes; the results being most pronounced at the shorter pulses.

A number of design approaches to investigate the effects of this principle have been made, including the use of stroboscopic light in small experimental cultures and rotating culture vessels, such as a rotating drum (described in Oswald et al. 1965, Proc. Am.Soc. Civil Eng. U(Sanit.Eng.), 91(SA4), 23) where the culture is kept as a thin film on the walls of the rotating drum by the centrifugal force, or as in the so called Couette device, where the culture is rotated in a narrow gap between a central rotor and an outer static wall. Ichimura (1976, United States Patent No 3,986,297) describes a non-translucent photosynthetic reactor tank where light is administered through dry wells in the tank. A flashing effect is obtained by rotating a shutter with a slit around the light source. Although the flashing light effect in this system may result in increased photosynthetic conversion of the actually emitted light, a similar overall effect can not be expected as the flashing essentially is brought about by shading the light source.

Neither of these systems have any production scale applications as the energy input and construction costs to establish and maintain such systems are not justified by the productivity increases.

A system inducing stratified turbulence in outdoor open race way tanks by the introduction of wing-like bafflers in the culture is described by Laws et al. 1983 Biotechnol. Bioeng.25,2319-2335; this system, however, is still structurally complex and can not be implemented in an indoor culture system as used as production cultures, producing for example micro algae for bivalve hatcheries and nurseries, marine fish hatcheries, and industrial micro algae applications, such as human nutritional products, pharmaceutical products etc.

Known production technology for these applications comprise either 50-500 litre transparent hard cylinders or plastic bags, open cubic or rectangular tanks, all illuminated and stirred in non-strategic manners using randomly injected air bubbles.

As used herein, the term "light" refers to electromagnetic radiation having wavelengths in the ultraviolet, visible and/or infrared regions of the electromagnetic spectrum.

US5,281,533 and US4,326,034 disclose known cultivation processes over which the invention is characterised.

In accordance with the present invention, apparatus for cultivating microorganisms comprises a vessel for containing microorganisms and a light source, the light source emitting light having a flux density greater than $500 \mu Em^{-2}sec^{-1}$.

Preferably, the apparatus further comprises a movement mechanism to generate relative movement between the light source and the microorganisms.

Typically, the movement mechanism comprises a stirring device which stirs the microorganisms in the vessel to generate the relative movement.

Typically, the stirring device may comprise an impeller type stirrer or an air lift stirrer mounted within the vessel.

Preferably, a number of light sources are provided. The light source or sources are typically arranged around the periphery of the vessel and are preferably outside the vessel and the vessel is substantially transparent to the light.

Typically, the light has a flux density of greater than $600 \mu Em^{-2}sec^{-1}$, and most preferably greater than $1000 \mu Em^{-2}sec^{-1}$.

Preferably, the light sources are spaced around the periphery and are arranged such that not all the periphery of the vessel is directly exposed to light; typically, the width of the light striking the vessel and the said relative movement is generated such that exposure of particular microorganisms in the vessel to light is not more than 2 seconds at a time.

Preferably, the light sources are equi-spaced around the periphery of the vessel.

Preferably, where the vessel is cylindrical, and a number of light sources "n" are provided with length "l" and width "b", and the length "l" extends substantially parallel to the central axis of the cylinder, then n times 1 is less than or equal to $\frac{1}{3}$ of the circumference of the cylinder.

Typically, the relative movement is relative rotation between microorganisms in the vessel and the light source, such that the rotations per minute is greater than $60b(d2\pi)$ where b is the width of the light source and d is the diameter of the vessel, and preferably n times b is less than $\pi d/4$ where n is the number of light sources.

An example of apparatus for cultivating microorganisms in accordance with the invention will now be described with reference to the accompanying drawings, in which:-

Fig. 1 is a schematic plan view of a bioreactor;
Fig. 2 is a schematic side view of the bioreactor shown in Fig. 1;
Figs. 3a and 3b are a plan view and a side view respectively of an air lift stirrer for use with the bioreactor shown in Figs. 1 and 2; and,
Figs. 4a and 4b are a plan view and a side view respectively of an impeller type stirrer for use with the bioreactor shown in Figs. 1 and 2.

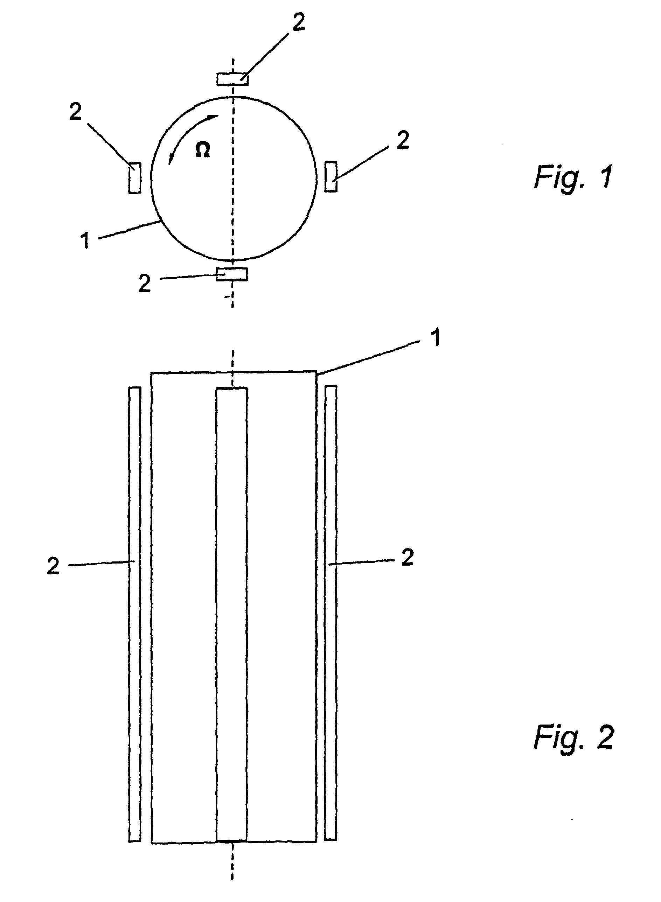

Fig. 1 shows a bioreactor which comprises a cylindrical vessel 1 in which microorganisms in the form of an algae culture is contained (see Figs. 1 and 2). Distributed around the outside of the vessel 1 are four light sources 2. Each light source 2 has a length l and a width b.

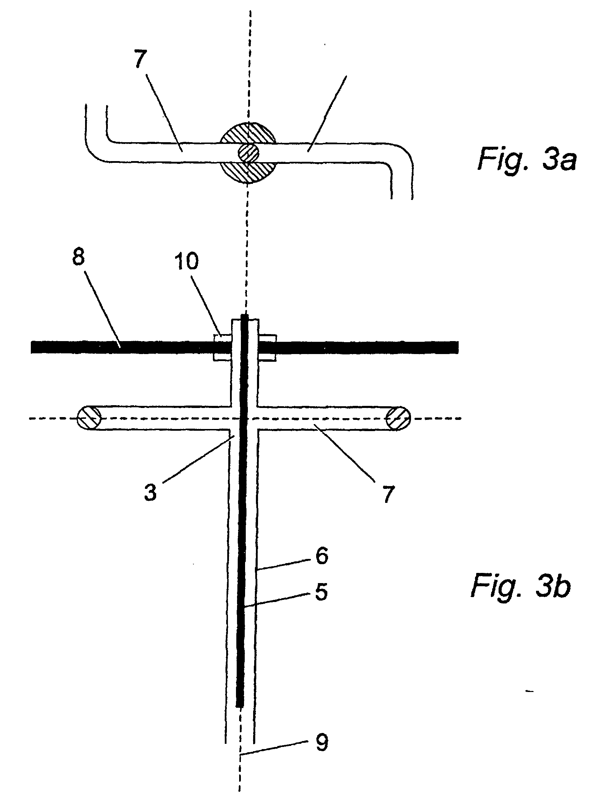

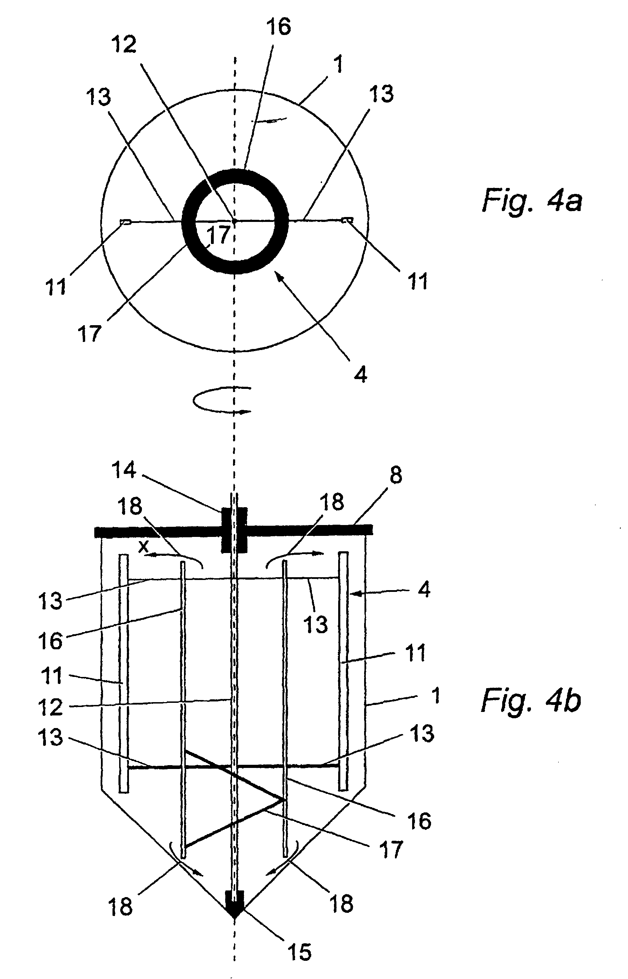

The algae within the vessel 1 are rotated by a stirrer mechanism at an angular speed of $\Omega$ revolutions per minute. The stirrer mechanism may be an air lift stirrer 3 (see Figs. 3a and 3b) or an impeller type stirrer 4 (see Figs. 4a and 4b).

Stirring of the culture contained in the cylindrical vessel 1 consists of two components:

1     rotation around the axis of the vessel 1; and,
2     a vertical displacement of the culture contained in the vessel 1.

The air lift stirrer 4 comprises a vertical tube 6 rotationally supported by a bearing 10 in a lid 8 of the vessel 1. Within the tube 6 is an air tube 5. In the air lift stirrer 3, as illustrated in Figs. 3a and 3b, air bubbles are ejected from the air tube 5 which extends almost to the bottom of the vertical tube 6. The culture fluid is lifted up the tube 6 from the bottom of the tube 6 and ejected through the two horizontal arms 7 in a tangential direction. The culture fluid in the vessel 1 is thereby rotated around a vertical axis 9 and is further more displaced in a vertical plug flow mode from top to bottom of the cylinder parallel to the axis 9.

In the impeller type stirrer 4, as illustrated in Figs. 4a and 4b, vertically oriented rectangular impellers 11 are mounted on a central shaft 12 by supporting rods 13. The shaft 12 extends through the lid 8 for example by a magnetically coupled bearing 14. The shaft 12 is supported distally by a lower bearing 15. An inner rotating cylinder 16 is fixed to shaft 12 also by the impeller support rods 13. Part of the inner surface of the cylinder 16 is covered with a helical fringe 17. Hence, the inner cylinder 16 acts as a screw pump when it is rotated to move the culture fluid through the inner cylinder 16. In Fig. 4b the fluid direction in the inner cylinder 16, as indicated by arrows 18, is in the direction bottom to top. However, the direction may be reversed.

The flashing light principle is implemented in the bioreactor shown in Figs. 1 and 2 by concentrating the light input in four vertical strands of light from the light sources 2, extending along preferably the entire culture-filled part of the cultivation cylinder.

The useful flux of visible light is 300 to $3000\mu Em^{-2}sec^{-1}$; preferably higher than $600\mu Em^{-2}sec^{-1}$ and most preferably higher than $1000\mu Em^{-2}sec^{-1}$.

The volumetric irradiation (visible light) dose is $0.12E/litre/day <I/d(24 \text{ hrs}) <0.36E/litre/day$.

Above $1000\mu Em^{-2}sec^{-1}$, an illuminated surface will be free of attached growth, from $600\mu Em^{-2}sec^{-1}$ to $1000\mu Em^{-2}sec^{-1}$, however, the attached growth problems are still greatly reduced.

Although four light sources 2 are shown, any number may be used. However, preferably two to four are used.

The incident light fields should be arranged as a number n of narrow strands each of length l and width b. The length l should cover a substantial part of the culture filled length of the vessel 1, preferably all of it. n x b should as small a fraction of the entire circumference of the cylinder as possible, and in all circumstances less than 25%.

The maximum circumference of the vessel 1 which should be covered by the light fields is given by the following equation:

$$n * b/d\pi <0.25$$

The duration of each light pulse should be less than 2 seconds and to achieve this the angular speed $\Omega$ (rpm) of the culture should be greater than $60b/(d2\pi)$.

By concentrating incident light in narrow patches with intensity above certain limits, wall growth of photosynthetic microorganisms in the reactor, which is a serious practical problem with previous types of photo bioreactors, is significantly reduced as the light intensity immediately below the wall of the culture cylinder is to high for attached biomass to be able to multiply and grow.

## Claims

1.  Apparatus for cultivating microorganisms comprising a vessel for containing microorganisms and a light source, the light source emitting light having a flux density greater than $500\mu Em^{-2}sec^{-1}$.

2.  Apparatus according to claim 1 having a movement mechanism to generate relative movement between the light source and the microorganisms.

3.  Apparatus according to claim 2, wherein the movement mechanism comprises a stirring device which stirs the microorganisms in the vessel to generate the relative movement.

4.  Apparatus according to claim 3, wherein the stirring device comprises an impeller-type stirrer or an air-lift stirrer mounted within the vessel.

5.  Apparatus according to any preceding claim, wherein a number of light sources are provided.

6.  Apparatus according to claim 5, wherein the light sources are spaced around the periphery of the vessel.

7.  Apparatus according to any preceding claim, wherein the light source(s) is outside the vessel.

8.  Apparatus according to any preceding claim, wherein the vessel is substantially transparent to the light.

9.  Apparatus according to any preceding claim, wherein the light has a flux density of greater than $600\mu Em^{-2} sec^{-1}$.

10. Apparatus according to any preceding claim, wherein the light has a flux density of greater than $1000\mu Em^{-2}sec^{-1}$.

11. Apparatus according to any preceding claim, wherein several light sources are arranged such that not all the periphery of the vessel is directly exposed to light.

12. Apparatus according to any preceding claim, wherein the width of the light striking the vessel and the said relative movement is generated such that the period of exposure of particular microorganisms in the vessel to light is not more than 2 seconds.

13. Apparatus according to any preceding claim, wherein the vessel is cylindrical.

14. Apparatus according to claim 13, wherein a number of light sources "n" are provided with length "1" and width "b", and their length "l" extends substantially parallel to the central axis of the cylinder, and wherein n x l is less than or equal to 1/3rd of the circumference of the cylinder.

15. Apparatus according to any preceding claim, wherein the relative movement is relative rotation between microorganisms in the vessel and the light source.

16. Apparatus according to claim 15, wherein the rotations per minute is greater than $60b(d2\pi)$ where b is the width of the light source and d is the diameter of the vessel.

17. Apparatus according to claim 16, wherein n x b is less than $\pi d/4$ where n is the number of light sources.

18. A method of cultivating microorganisms in a culture vessel, the method comprising illuminating the culture vessel with at least one light source, the light source emitting light having a flux density greater than $500 \mu Em^{-2}sec^{-1}$.

19. A method according to claim 18, wherein the method includes the further step of moving the microorganisms relative to the light source.

20. A method according to claim 18 or 19, wherein the method includes the step of illuminating the microorganisms from outwith the culture vessel.

21. A method according to claim 20, wherein the microorganisms are illuminated from outside the culture vessel which is substantially transparent to the light.

22. A method according to anyone of claims 18-21, the method comprising providing a number n of light sources of length l along the length of a cylindrical vessel wherein n x l is less than or equal to 1/3rd of the circumference of the cylinder, and wherein a unit volume of the contents adjacent the periphery of the cylinder is subjected to a regime of intermittent light and dark periods.

**Patentansprüche**

1. Eine Vorrichtung zur Kultivierung von Mikroorganismen, bestehend aus einem Behälter zur Aufnahme von Mikroorganismen und einer Lichtquelle, wobei die Lichtquelle Licht mit einer Flussdichte von mehr als 500 $\mu Em^{-2}sec^{-1}$ ausstrahlt.

2. Vorrichtung gemäß Anspruch 1 mit einem Bewegungsmechanismus zur Erzeugung von Relativbewegung zwischen der Lichtquelle und den Mikroorganismen.

3. Vorrichtung gemäß Anspruch 2, wobei der Bewegungsmechanismus aus einem Rührapparat besteht, der die Mikroorganismen in dem Behälter rührt, um die Relativbewegung zu erzeugen.

4. Vorrichtung gemäß Anspruch 3, wobei der Rührapparat aus einem Schnellrührer oder einem Druckluftrührer besteht, der innerhalb des Behälters angebracht ist.

5. Vorrichtung gemäß einem der vorhergehenden Ansprüche, wobei eine Anzahl von Lichtquellen bereitgestellt wird.

6. Vorrichtung gemäß Anspruch 5, wobei die Lichtquellen in Abständen um den Umfang des Behälters angeordnet sind.

7. Vorrichtung gemäß einem der vorhergehenden Ansprüche, wobei sich die Lichtquelle(n) außerhalb des Behälters befindet/befinden.

8. Vorrichtung gemäß einem der vorhergehenden Ansprüche, wobei der Behälter im Wesentlichen lichtdurchlässig ist.

9. Vorrichtung gemäß einem der vorhergehenden Ansprüche, wobei das Licht eine Flussdichte von mehr als 600 $\mu Em^{-2}sec^{-1}$ aufweist.

10. Vorrichtung gemäß einem der vorhergehenden Ansprüche, wobei das Licht eine Flussdichte von mehr als 1000 $\mu Em^{-2}sec^{-1}$ aufweist.

11. Vorrichtung gemäß einem der vorhergehenden Ansprüche, wobei mehrere Lichtquellen so arrangiert sind, dass nicht der ganze Umfang des Behälters direkt belichtet wird.

12. Vorrichtung gemäß einem der vorhergehenden Ansprüche, wobei die Breite des Lichts, das auf den Behälter trifft, und die Relativbewegung so erzeugt werden, dass die Belichtungszeit bestimmter Mikroorganismen in dem Behälter nicht mehr als zwei Sekunden beträgt.

13. Vorrichtung gemäß einem der vorhergehenden Ansprüche, wobei der Behälter zylindrisch ist.

14. Vorrichtung gemäß Anspruch 13, wobei eine Anzahl von Lichtquellen "n" mit Länge "I" und Breite "b" versehen werden und ihre Länge "I" sich im Wesentlichen parallel zur Zentralachse des Zylinders erstreckt, und wobei n x I kleiner/gleich einem Drittel des Umfangs des Zylinders ist.

15. Vorrichtung gemäß einem der vorhergehenden Ansprüche, wobei die Relativbewegung Relativdrehung zwischen Mikroorganismen in dem Behälter und der Lichtquelle ist.

16. Vorrichtung gemäß Anspruch 15, wobei die Drehungen pro Minute mehr als 60b(d2π) betragen, wobei b die Breite der Lichtquelle ist und d der Durchmesser des Behälters ist.

17. Vorrichtung gemäß Anspruch 16, wobei n x b weniger als πd/4 beträgt, wobei n die Anzahl von Lichtquellen ist.

18. Ein Verfahren zur Kultivierung von Mikroorganismen in einem Kulturbehälter, wobei das Verfahren aus dem Beleuchten des Kulturbehälters mit mindestens einer Lichtquelle besteht, wobei die Lichtquelle Licht mit einer Flussdichte von mehr als 500 $\mu Em^{-2}sec^{-1}$ ausstrahlt.

19. Verfahren gemäß Anspruch 18, wobei das Verfahren den weiteren Schritt beinhaltet, die Mikroorganismen relativ zur Lichtquelle zu bewegen.

20. Verfahren gemäß Anspruch 18 oder 19, wobei das Verfahren den Schritt beinhaltet, die Mikroorganismen von außerhalb des Kulturbehälters zu beleuchten.

21. Verfahren gemäß Anspruch 20, wobei die Mikroorganismen von außerhalb des Kulturbehälters, der im Wesentlichen lichtdurchlässig ist, beleuchtet werden.

22. Verfahren gemäß einem der Ansprüche 18 - 21, wobei das Verfahren aus dem Bereitstellen einer Anzahl n von Lichtquellen der Länge I entlang der Länge eines zylindrischen Behälters besteht, wobei n x I kleiner/gleich einem Drittel des Umfangs des Zylinders ist, und wobei ein Einheitsvolumen der Inhalte, das an dem Umfang des Zylinders anliegt, einem Bereich diskontinuierlicher heller und dunkler Zeitabschnitte ausgesetzt wird.

## Revendications

1. Appareil de culture de microorganismes comprenant un récipient destiné à contenir des microorganismes et une source lumineuse, la source lumineuse émettant de la lumière ayant une densité de flux supérieure à 500 $\mu Em^{-2}sec^{-1}$.

2. Appareil selon la revendication 1 possédant un mécanisme de déplacement pour produire un déplacement relatif entre la source lumineuse et les microorganismes.

3. Appareil selon la revendication 2, dans lequel le mécanisme de déplacement comprend un dispositif d'agitation qui agite les microorganismes dans le récipient pour produire un déplacement relatif.

4. Appareil selon la revendication 3, dans lequel le dispositif d'agitation consiste en un agitateur de type à palette ou un agitateur à air monté au sein du récipient.

5. Appareil selon n'importe quelle revendication précédente, dans lequel il est prévu un certain nombre de sources lumineuses.

6. Appareil selon la revendication 5, dans lequel les sources lumineuses sont espacées autour de la périphérie du récipient.

7. Appareil selon n'importe quelle revendication précédente, dans lequel la (les) source(s) lumineuse(s) se trouve(nt) à l'extérieur du récipient.

8. Appareil selon n'importe quelle revendication précédente, dans lequel le récipient est sensiblement transparent à la lumière.

9. Appareil selon n'importe quelle revendication précédente, dans lequel la lumière a une densité de flux supérieure à 600 $\mu Em^{-2}\ sec^{-1}$.

10. Appareil selon n'importe quelle revendication précédente, dans lequel la lumière a une densité de flux supérieure à 1 000 $\mu Em^{-2}\ sec^{-1}$.

11. Appareil selon n'importe quelle revendication précédente, dans lequel plusieurs sources lumineuses sont agencées de telle sorte que la périphérie du récipient ne soit pas toute directement exposée à la lumière.

12. Appareil selon n'importe quelle revendication précédente, dans lequel la largeur de la lumière frappant le récipient et ledit déplacement relatif sont produits de telle sorte que la durée d'exposition à la lumière de microorganismes particuliers dans le récipient ne dépasse pas 2 secondes.

13. Appareil selon n'importe quelle revendication précédente, dans lequel le récipient est cylindrique.

14. Appareil selon la revendication 13, dans lequel il est prévu un nombre de sources lumineuses "n" possédant une longueur "l" et une largeur "b", leur longueur "l" se prolongeant de façon sensiblement parallèle à l'axe central du cylindre, et dans lequel n x l est inférieur ou égal à 1/3 de la circonférence du cylindre.

15. Appareil selon n'importe quelle revendication précédente, dans lequel le déplacement relatif est la rotation relative entre des microorganismes dans le récipient et la source lumineuse.

16. Appareil selon la revendication 15, dans lequel la rotation par minute est supérieure à 60b(d2π) où b est la largeur de la source lumineuse et d est le diamètre du récipient.

17. Appareil selon la revendication 16, dans lequel n x b est inférieur à πd/4 où n est le nombre de sources lumineuses.

18. Un procédé de culture de microorganismes dans un récipient de culture, le procédé comprenant l'illumination du récipient de culture avec au moins une source lumineuse, la source lumineuse émettant de la lumière ayant une densité de flux supérieure à 500 $\mu Em^{-2}sec^{-1}$.

19. Un procédé selon la revendication 18, dans lequel le procédé comporte l'étape supplémentaire consistant à déplacer les microorganismes par rapport à la source lumineuse.

20. Un procédé selon la revendication 18 ou la revendication 19, dans lequel le procédé comporte l'étape consistant à illuminer les microorganismes depuis le dehors du récipient de culture.

21. Un procédé selon la revendication 20, dans lequel les microorganismes sont illuminés de l'extérieur du récipient de culture, lequel est sensiblement transparent à la lumière.

22. Un procédé selon n'importe laquelle des revendications 18 à 21, le procédé consistant à fournir un nombre n de sources lumineuses de longueur l sur la longueur d'un récipient cylindrique, dans lequel n x l est inférieur ou égal à 1/3 de la circonférence du cylindre, et dans lequel un volume unitaire du contenu adjacent à la périphérie du cylindre est soumis à un régime de périodes lumineuses et sombres intermittentes.

*Fig. 1*

*Fig. 2*

7

*Fig. 3a*

8    10

3        7

6

5

*Fig. 3b*

9

Fig. 4a

Fig. 4b

9